# EUROPEAN PATENT SPECIFICATION

(11) **EP 2 050 455 B1**
(45) Date of publication and mention of the grant of the patent: **01.08.2012**
(21) Application number: 07020351.8
(22) Date of filing: 18.10.2007
(51) Int. Cl.: A61K 31/715, A61K 38/08, A61P 35/00, A61P 37/04

(54) **Compounds leading to an increase of the IL-12/IL-10 ratio and their use for therapy of infectious and proliferative diseases**
Verbindungen zur Erhöhung des IL-12/IL-10-Verhältnisses und ihre Verwendung bei der Therapie infektiöser und proliferativer Krankheiten
Composants menant à une augmentation du rapport IL-12/IL-10 et leur utilisation pour la thérapie des maladies infectieuses et proliférantes

(43) Date of publication of application: 22.04.2009
(73) Proprietor: DC Gesellschaft für dentritic-cell-Therapie mbH, 37075 Göttingen (DE)
(72) Inventor: Lorenzen, Dirk, 37136 Waake (DE); Nesselhut, Thomas, 37075 Göttingen (DE); Peters, J. Hinrich, 37077 Göttingen (DE); Wiesmüller, Karl-Heinz, 72070 Tübingen (DE); Freudenberg, Marina A., 79108 Freiburg (DE); Galanos, Chris, 79194 Gundelfingen (DE); Bessler, Wolfgang G., 79194 Gundelfingen (DE)
(74) Representative: Schüssler, Andrea

(56) References cited:
- KEESTRA A M ET AL: "The central leucine-rich repeat region of chicken TLR16 dictates unique ligand specificity and species-specific interaction with TLR2" JOURNAL OF IMMUNOLOGY 01 JUN 2007 UNITED STATES, vol. 178, no. 11, 1 June 2007 (2007-06-01), pages 7110-7119, XP002468538 ISSN: 0022-1767
- OKUSAWA T ET AL: "Relationship between Structures and Biological Activities of Mycoplasmal Diacylated Lipopeptides and Their Recognition by Toll-Like Receptors 2 and 6" INFECTION AND IMMUNITY 2004 UNITED STATES, vol. 72, no. 3, 2004, pages 1657-1665, XP002468539 ISSN: 0019-9567
- KIURA KAZUTO ET AL: "The diacylated lipopeptide FSL-1 induces TLR2-mediated Th2 responses." FEMS IMMUNOLOGY AND MEDICAL MICROBIOLOGY OCT 2006, vol. 48, no. 1, October 2006 (2006-10), pages 44-55, XP002468540 ISSN: 0928-8244
- HASHIMOTO M ET AL: "Evidence of immunostimulating lipoprotein existing in the natural lipoteichoic acid fraction" INFECTION AND IMMUNITY 2007 UNITED STATES, vol. 75, no. 4, April 2007 (2007-04), pages 1926-1932, XP002468541 ISSN: 0019-9567
- KIURA KAZUTO ET AL: "The synthetic analogue of mycoplasmal lipoprotein FSL-1 induces dendritic cell maturation through Toll-like receptor 2." FEMS IMMUNOLOGY AND MEDICAL MICROBIOLOGY FEB 2006, vol. 46, no. 1, February 2006 (2006-02), pages 78-84, XP002468542 ISSN: 0928-8244

## Description

The present invention relates to the treatment of immunologically relevant diseases such as infections or proliferative diseases, in particular cancer, by increasing the IL-12/IL-10 ratio using the sequential administration of lipopeptides and lipopolysaccharides alone or in combination with IFN-γ.

Dendritic Cells (DC): DC are the initiator cells of the specific immune reaction. They are central in inducing T-helper lymphocytes, which later then induce mainly T-lymphocytes, but also B-lymphocytes. Consequently, DC can be used as vehicles to transport antigens into the immune system. This can be utilized to induce a prophylactic or a therapeutic vaccination against the antigen(s) DC have been exposed to. DC do not only act as stimulators of the immune cascade, instead they are also the source of immune suppression and of immune tolerance induction. Which of these antagonistic pathways will actually be induced is a matter of immune regulation. This is reflected by different states of differentiation and activation of DC. Most of these various stages of DC development are exogenously induced by ligands, cytokines, microbial agents, tumor cell products and others.

Myeloid dendritic cells: Myeloid DC segregate from various stages of development, thus they can also be derived from monocytes. In the periphery of the organism myeloid DC are known under different names such as "Langerhans cells" of the skin or "interdigitating reticulum cells" within the lymphoid organs. They derive from the myeloid lineage of bone marrow cells, which within the haematopoiesis give rise to monocytes, macrophages and granulocytes. They first develop into a state of "immature DC", also designated as "sentinel DC", bearing the function of antigen uptake, -processing and -presentation. In order to be able to successfully present these antigens to the responding T-lymphocytes, specifically programmed against these antigens, DC have to further develop or mature to the state of "mature" or "effector" DC.

During the process of maturation a branching, "polarization", is possible into at least two directions, designated as Th1 and Th2 directions, and the corresponding DC are termed DC1 and DC2, respectively. They imply different types of immune stimulation.

Monocyte-derived immature DC (iMoDC): iMoDC are typically prepared from monocytes of the peripheral blood. Monocytes are isolated by various methods such as density and velocity gradient centrifugation followed by selective attachment and washing away of the non-attached cells, mainly lymphocytes. Other methods are leukapheresis followed by elutriation (centrifugal counterflow elutriation) or magnetically sorted CD14⁺ cells using the MACS-system (Miltenyi Biotec). Monocytes are treated with respective inducers under cell culture conditions to differentiate them into immature DC, mostly by using GM-CSF plus IL-4. Alternatively, IL-13 instead of IL-4 can be used. Other inducers such as calcium ionophore have also been published to generate MoDC. After 5-7 days of differentiation the state of immature DC is reached. They are now prepared to take up added antigens, to process and to present it in the context of MHC molecules on the cell surface.

Maturation: Maturation is induced by uptake of antigen but further stimulated by various triggers. iDC pass through a process of "maturation", typically finished after 2 days. They have a reduced capability of taking up antigens, but instead they have reached the ability to stimulate T-lymphocytes. Phenotypically they are characterized by the increased expression of costimulatory molecules such as CD80 and CD86 as well as expression of the maturation marker CD83. IL-12 and IL-10 will be secreted, an activity that will be exhausted depending on the time and the strength of stimulation. The IL-12/IL-10 ratio will not significantly be influenced.

Standard-inducers of DC-maturation are a cytokine-cocktail ("maturation cocktail", a widely used standard cocktail, TNF-alpha, IL-1beta, IL-6, PEG2 according to Jonuleit et al., Eur J Imnunol 27(12): 3135-42 (1997)), CD40-ligand (simulating the interaction with T-lymphocytes and IFN-γ which is involved into the feedback-loop of IL-12 production and therefore combined with several maturation and polarizing factors, mainly favouring a Th1 direction).

Polarization: As already mentioned above, during maturation DC may branch into alternating functional directions, designated as "polarization". Again, polarization is exogenously controlled. Main directions of polarization are Th1, leading to generation of cytotoxic T-lymphocytes, and Th2, leading mainly to activation of B-lymphocytes, but also to immune suppression and tolerance induction. Th1 direction is responsible for defense against tumors and microorganisms. It is characterized by the production of IFN-γ. Release of IL-12 by DC is a precondition for production of IFN-γ. Performing one direction to dominate the alternative direction is performed by the mechanism of cross-regulation: cytokines secrete to stimulate one direction are concomitantly inhibitory for the other direction and *vice versa*.

Immune escape: Pathogenic microorganisms as well as tumors have developed strategies to undermine the immune response. They have the goal to trigger the immune reaction into a direction ineffective against the pathogen. This makes the pathogen successful to survive and the disease will not be defended. An active vaccination against the disease has to consider these immune escape mechanisms. Consequently, DC have to be armed to resist these irritations. Such, the envisaged state of DC has to be actively induced, even against the pressure of counteracting factors. Within the actual scenario of anti-tumor as well as anti-microbial defense it is the concrete goal to obtain the state of Th1 polarisation. Functionally, this is characterized by a functional dominance of IL-12 over IL-10. Thus, any increase of the IL-12/IL-10 ratio is advantageous.

Extracellular signals: As mentioned, any state of differentiation, maturation and polarization of DC can be induced exogenously. Conversely, if exogenous stimuli are missing, the DC are not only arrested in the present state but rather fall back into a state rendering them immunosuppressive and even tolerance inducing. Extracellular signals are constituted either from endogenous factors from the organism such as cytokines, hormones and components of the extracellular matrix, or foreign agents such as foreign bodies and antigens, but mainly microbes or microbial components. Consequently, endogenous as well as exogenous factors can be utilized as inducers for an intended direction of DC triggering.

Interleukin-12 (IL-12) and interleukin-10 (IL-10): The desired anti-microbial and anti-tumor activity requires a Th1 polarisation. On the level of DC this is characterized by secretion of IL-12. IL-12 is antagonized by Il-10, which is also produced by DC. Therefore, the envisaged profile of DC activity is characterized by a high IL-12 production and release, accompanied by a low IL-10 production and release, expressed by the IL-12/IL-10 ratio. IL-12 is produced by dendritic cells, monocytes, macrophages, and B-lymphocytes. It acts synergistically with IL-2 to induce differentiation of resting T-cells into cytotoxic T-lymphocytes. It stimulates proliferation of NK and LAK cells.

IL-10 is produced by Th2 cells, regulatory T-cells and Dendritic Cells. It acts on macrophages, DC and other antigen-presenting cells. It inhibits cytokine production and thus indirectly reduces cytokine production by Th1 cells. It down-regulates class II MHC molecule expression on antigen-presenting cells and also inhibits the DC maturation.

Microbial inducers: The immune system, phylogenetically developed against invasive microorganisms, has developed a number of prepared defense structures, ranging from single secreted molecules to cellular receptors. These ligands are designed to bind and to neutralize the pathogens or their toxins. In the co-evolution the microorganisms have developed increasingly complicated structures in order to circumvent the defence. Some of them are pyrogens which induce fever or are even toxic (endotoxins). These molecules, basing on a basic structure, are highly diverse and are, in a general term, designated as Pathogen-associated molecular patterns (PAMPs). Main groups are lipopeptides and lipopolysaccharides, most of them deriving from Gram-negative bacteria; see, e.g., Seya et al., Int J Biochem Cell Biol 34 (8) : 901-6 (Review on the lipoproteins from mycoplasms and their action on PAMS).

Toll-like receptors (TLR): Extracellular signals are converted into intracellular signals by membrane receptors. Some of them are directly designed as receptors for exogenous signals of microorganisms, namely Toll-like receptors. At present 11 TLR have been differentiated, directed against different microbial molecules. They activate various intracellular signalling pathways. They either act synergistically or antagonistically. TLR2 appears as single chain or, alternatively, as a heterodimer either with TLR1 or with TLR6. Some well-defined ligands for TLR are dsRNA or Poly-I:C (ligands of TLR3), CpG-derivatives (binding TLR9) and lipopolysaccharide (binding predominantly to TLR4). Bacterial lipopeptides bind, depending on their basic structure, either to the TLR-pair TLR1/2 (Triacyl-Lipopeptide), or to TLR2/6 (Diacyl-Lipopeptide).

Keestra A. M. et al. (J. Immunology, vol. 178, no. 11, 2007, p. 7110-7119) discloses the use of diacyl-lipopetide FSL-1 for stimulating the immune system. Okusawa T et al. (Infection and Immunity, vol. 72, no. 3, 2004, p. 1657-1665) describes the role of lipopolysaccharide and lipopeptides, i.e. FSL-1, in the activation of macrophages, fibroblasts or lymphocytes to induce production of cytokines. Kiura Kazuto et al. (FEMS Immunology and Medical Microbiolyogy, vol. 48, no. 1, 2006, p. 44-55) describes a mechanism of activation of the immune response by the recognition of lipopolysaccharides and lipopeptides through TLR activation. TLR4 activation by lipopolysaccharide induces IL-12p70 responses and IFN-γ production, while TLR2 stimulation only produces small amounts of IL-12p70. Further it is described that FSL-1 activates bone-marrow dendritic cells to stimulate production of IL-12p70 in a TLR2 dependent manner to induce a Th2 type of responses.

Danger signals: Specific recognition of an antigen has appeared not to be sufficient to mount a defence reaction against it. Instead, when additional signals are missing, immune suppression or tolerance will be induced. Additional signals required to establish a complete defence reaction are so called danger signals. Most of the common danger signals are derived from microbial components, particularly from pathogenic bacteria, known also as pyrogens and endotoxins. Danger signals influence many of the above mentioned effects on differentiation, maturation and polarisation of DC development. Thus, they represent a principle superimposed on the single steps of DC triggering. Danger signals lead to an increased expression of molecules of the MHC complex and of costimulatory molecules. Both result in an increased stimulation of T-lymphocytes.

Both, cytokines and microbial components induce a multiplicity of effects on the immune system. IFN-γ may in part be successful in increasing the IL-12/IL-10 ratio, especially in healthy control individuals. However, in tumor patients which exert a high immune suppressive effect of IL-10, VEGF and others, IFN-γ is little effective when added solely. None of the single agents has been found to separately upregulate IL-12 or to downregulate IL-10 without additional side effects. Moreover, none of them exerts a combined effect of upregulating IL-12 and downregulating IL-10 simultaneously. Thus, the technical problem underlying the present invention is to provide means upregulating IL-12 and downregulating IL-10 simultaneously.

The solution of the said technical problem is achieved by providing the embodiments characterized in the claims. The present invention is based on the findings obtained when immature DC were treated with a sequential addition of lipopeptide and a subsequent administration of lipopolysaccharide (alone or in the presence of IFN-γ). Surprisingly, the combined action resulted in an increase of the IL-12/IL-10 ratio.

### Brief description of the drawings

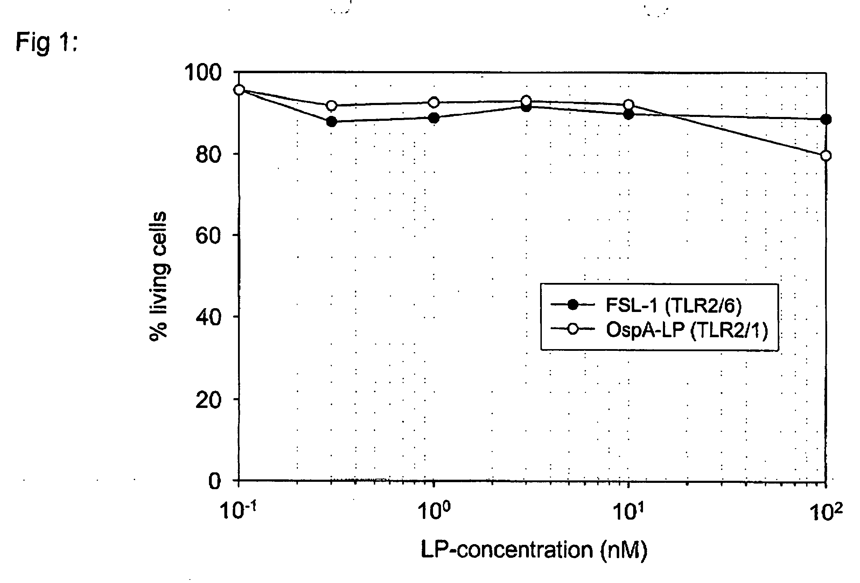

Figure 1: Cell viability under lipopeptide treatment
   Immature monocyte-derived dendritic cells (iMoDC), day 5, were treated for another 48 hours by different concentrations (abscissa) of the lipopeptides FSL-1 (ligand to TLR2/6) or OspA-LP, (ligand to TLR2/1), respectively. The number of living cells was measured by Casy-technology. As shown the viability remains high up to 100 nM.

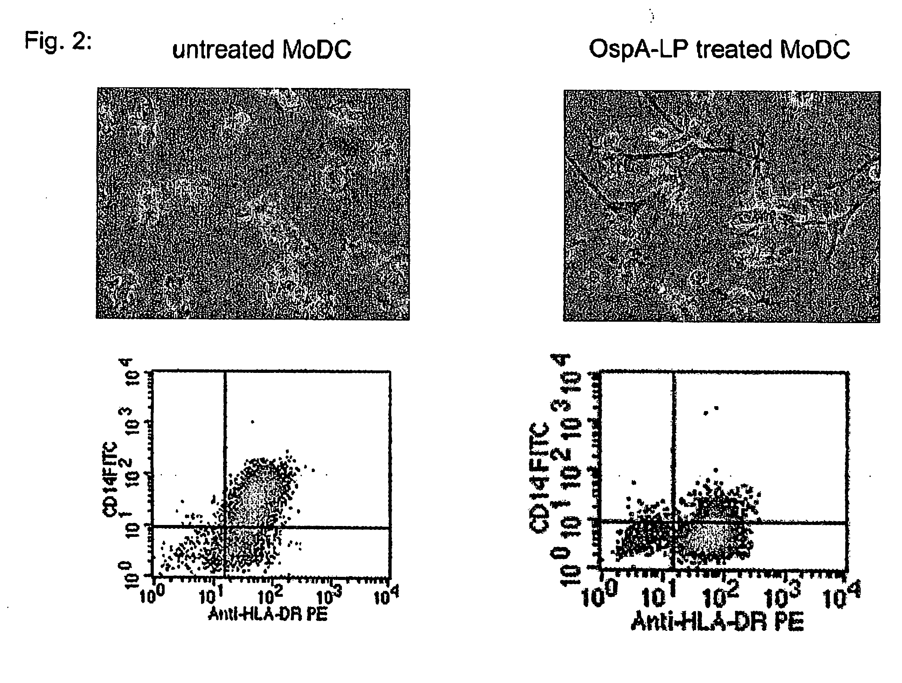

Figure 2: Phenotype modification by lipopeptide treatment
   iMoDC were treated with OspA-LP (10 nM) for 24 hours (right) or were left untreated (left). They were analysed for expression of CD14 versus HLA-DR. Morphology was assessed by phase contrast microscopy. Untreated MoDC (left) showed the typical veils and low expression of CD14. In contrast, OspA-LP treated MoDC expressed long cellular elongations and further downregulated CD14.

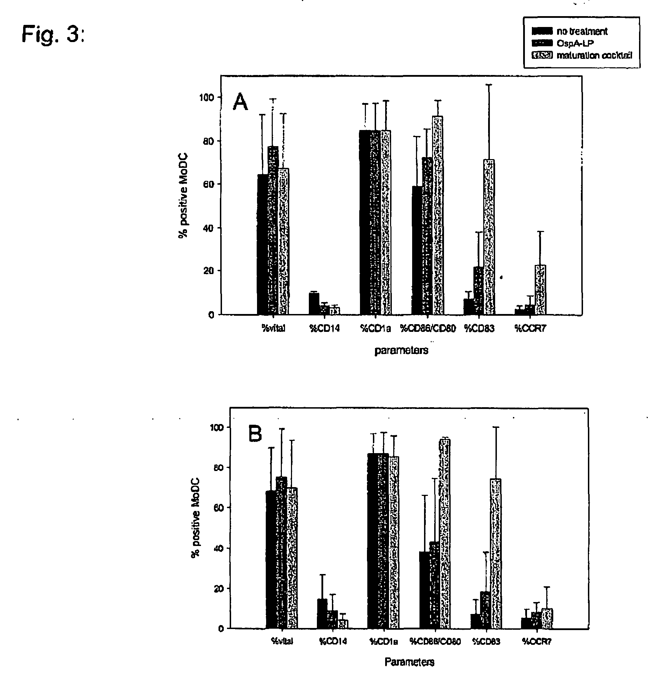

Figure 3: Effect of lipopeptide OspA-LP on the expression of surface molecules on MoDC.
   MoDC were cultured in serum containing media **(A)** or serum free **(B).** They were left untreated (black), treated with maturation cocktail as positive control (green), or with 10 nM OspA-LP (red) for 48 hours and analysed by flow cytometry for viability or the expression of CD14, CD1a, CD86/CD80 double positive cells, CD83 and CD197 (CCR7). Data were collected from 4 independent experiments. The results are means ±SD. It is shown that OspA acts as an incomplete maturation signal by upregulating CD80/CD86 but only modestly stimulating CD83 expression, as compared to the standard maturation mix. Other markers tested remain unaffected.

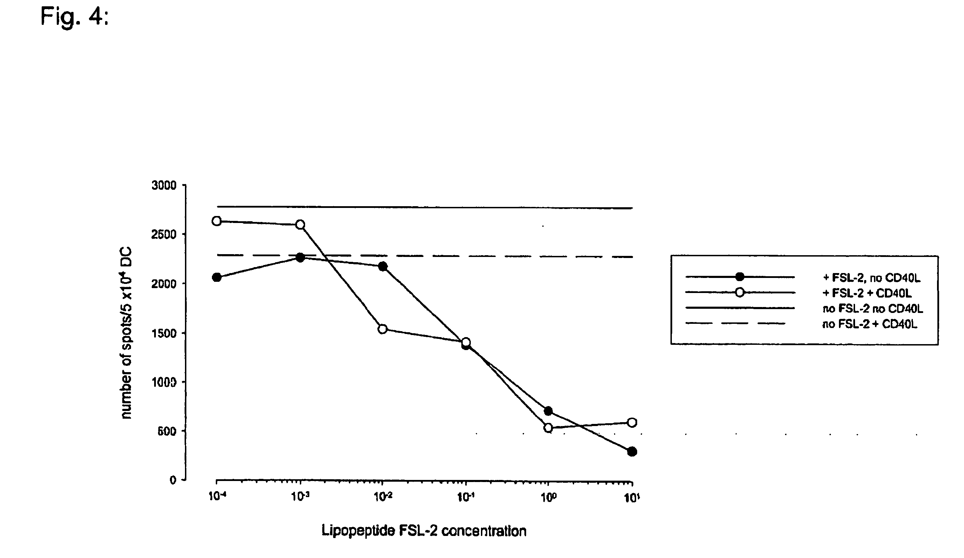

Figure 4: Dose-dependent down regulation of IL-10 production by lipopeptide FSL-2.
   iMoDC were cultured under serum free conditions and treated at day 6 either with lipopeptide FSL-2 alone or in combination with CD40 ligand (CD40L). After another 24 hours the cells were analysed for IL-10 production by ELISPOT assay. It is shown that at both conditions the lipopeptide FSL-2 dose-dependently suppresses IL-10-production.

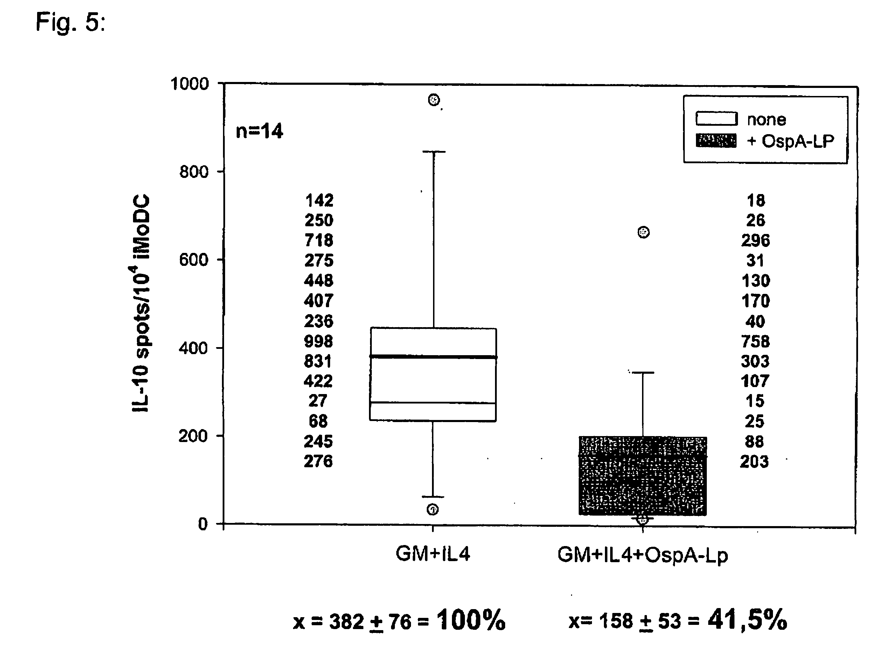

Figure 5: Donor-dependent variation of IL-10 production and its suppression by OspA-LP
   iMoDC from 14 healthy donors were tested for their basic IL-10 production (left) or the downregulated IL-10 production after treatment with 10 nM OspA-LP for 48 hours (right). The resulting values are given numbers of spots. Statistics are expressed as box plots with 95% confidential values; means (blue) and median values (black). x = mean value.

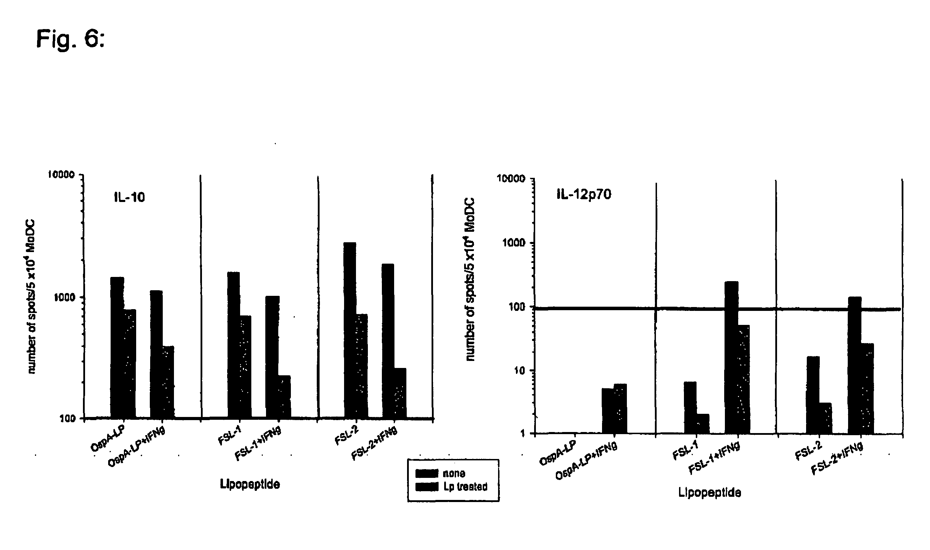

Figure 6: Lipopeptide treatment of MoDC reduces the number of IL-10 producing cells but is a poor inducer of IL-12p70 production
   iMoDC were treated either with the lipopeptide OspA-LP (10 nM), FSL-1 (1 nM) or FSL-2 (1 nM) alone or combined with IFN-γ (500 IU/ml) for 24 hours and tested for IL-10 production (left) and IL-12-p70 production (right). All LP treated MoDC (red) were compared with untreated MoDC (black). The blue line represents 100 spots per 5x10⁴ MoDC. It is shown that IL-10 is downregulated in all tested' conditions, whereas IL-12p70 remains extremely low.

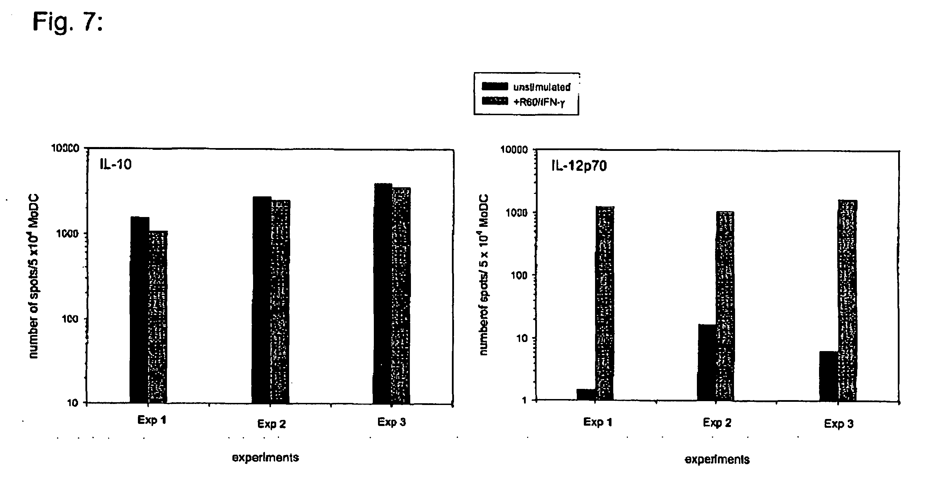

Figure 7: Effect of stimulation by LPS R60+IFN-γ on IL-10 and IL-12p70 production of MoDC
   iMoDC were treated with LPS R60 (1 µg/ml) combined with IFN-γ (500 IU/ml) for 24 hours. They were analysed for IL-10 (left) and IL-12p70 production (right). Shown are 3 independent experiments using cells from different healthy donors.
   Compared to the untreated controls (black), IL-10 production is not depressed by LPS R60/IFN-γ (red). IL-12p70 production is enhanced by the treatment.

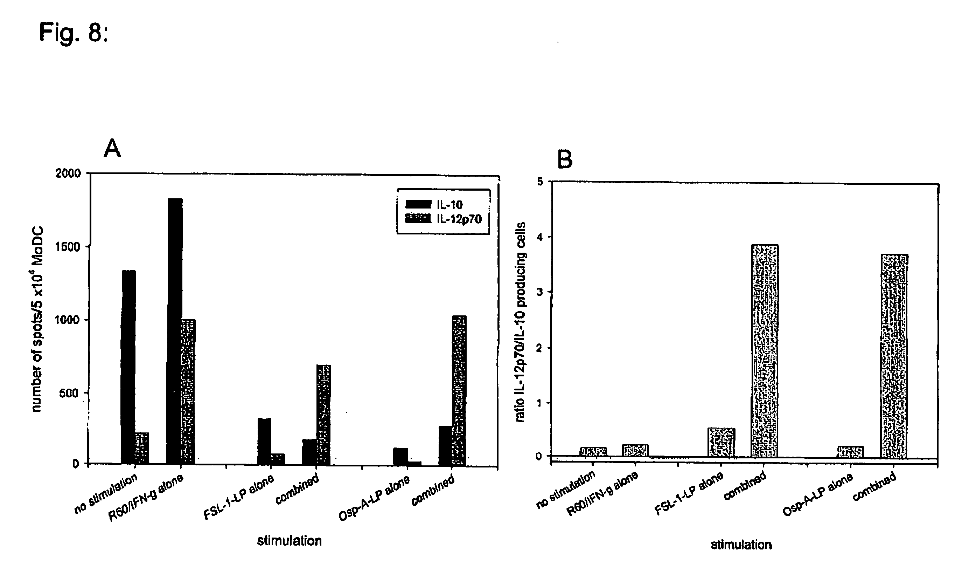

Figure 8: Effects of LPs FSL-1, OspA-LP and LPS R60/IFN-γ, and repective combinations on IL-10 and IL-12p70 production of MoDC
   MoDC were treated in a sequential manner first with a lipopeptide and after 15 hours with LPS R60/IFN-γ (1 µg/ml / 500 IU/ml). **(A)** number of cytokine producing cells: IL-10 (black); IL-12p70 (red). **(B)** ratio of IL-12p70/IL-10 producing cells.
   Only the combined treatment by lipopeptide and LPS R60/IFN-γ induced a Th1 polarising MoDC with low IL-10 and high IL-12p70 production.

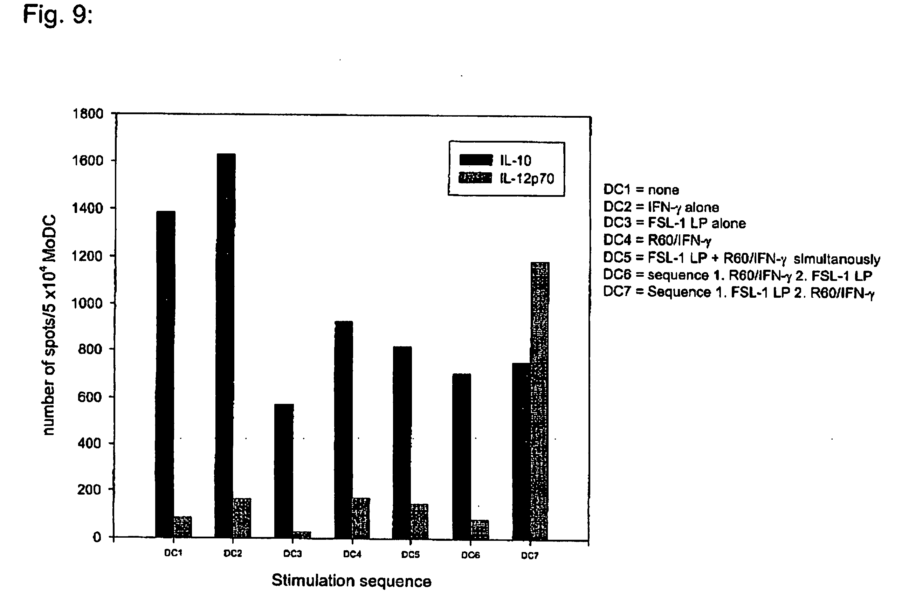

Figure 9: Combinations and sequential double treatment
   iMoDC were treated in 7 different combinations of three components: IFN-γ alone (500 IU/ml), FSL-1 LP (1 nM), and LPS R60/IFN-γ (1 µg/ml / 500 IU/ml). It is shown that only MoDC stimulated with combination 7 (LP followed by LPS) favoured IL-12p70 production.

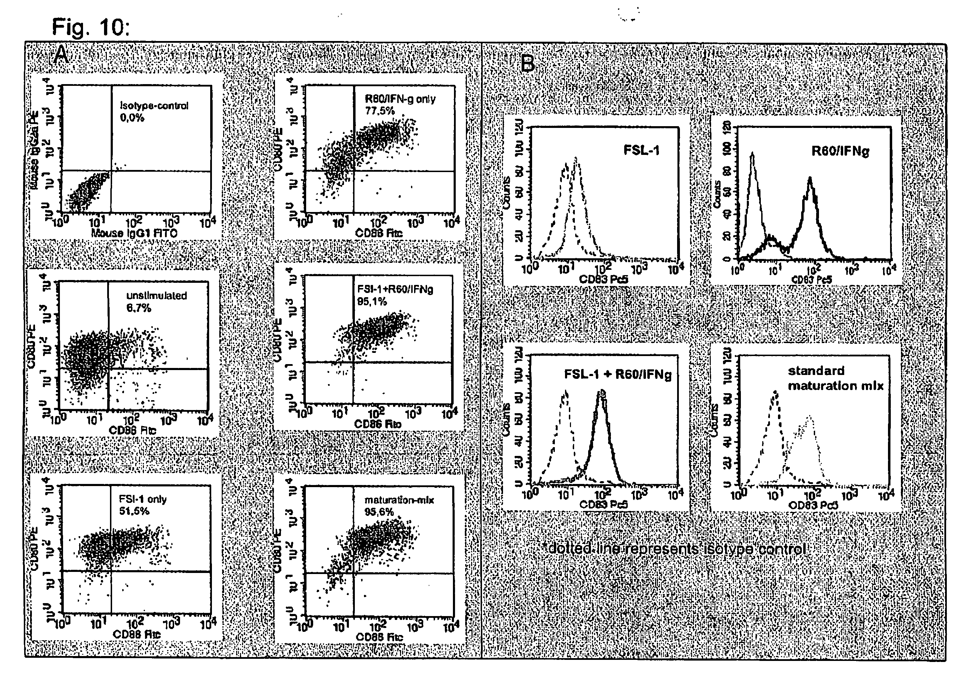

Figure 10: Maturation of MoDC as shown by surface markers Maturation of MoDC was induced by a standard protocol, by single additions of FSL-1 LP (1 nM) or LPS R60 (1 µg/ml)/IFN-γ (500 IU/ml) or their sequential combinations. The resulting cells were analysed for the surface expression of both CD80 and CD86 in dot plot analyses **(A)**, and CD83 histogram overlay **(B)**. It is shown that the lipopeptide induces only incomplete maturation (as shown by low expression of CD83), whereas maturation is induced by LPS-R60/IFN-γ, combination of FSL-1 with LPS-R60/IFN-γ and the standard maturation cocktail.

Accordingly, the present invention relates to a pharmaceutical composition containing (a) a ligand to TLR2 and (b) a ligand to TLR4 as separate entities according to claim 1.

Preferably, in said pharmaceutical composition the ligand to TLR2 and the ligand to TLR4 are present in an effective dose and combined with a pharmaceutically acceptable carrier. "Pharmaceutically acceptable" is meant to encompass any carrier, which does not interfere with the effectiveness of the biological activity of the active ingredients and that is not toxic to the host to which it is administered. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at an effective dose.

An "effective dose" refers to amounts of the active ingredients that are sufficient to affect the course and the severity of the disease, leading to the reduction or remission of such pathology.

Administration of the ligands may be effected by different ways, e.g. by intravenous, intratumoral, intranodal, intraperitoneal, subcutaneous, intramuscular, topical or intradermal administration. The route of administration, of course, depends on the kind of therapy and the kind of compounds contained in the pharmaceutical composition. A preferred route of administration is intravenous or intradermal administration. The dosage regimen will be determined by the attending physician and other clinical factors. As is well known in the medical arts, dosages for any one patient depends on many factors, including the patient's size, body surface area, age, sex, the particular compounds to be administered, time and route of administration, the kind of therapy, general health and other drugs being administered concurrently. Alternatively, DC will be treated during the time ex-vivo in cell culture, i.e. outside the body of the patient. For this purpose, the agents will be added to the culture in typical concentrations as mentioned in the legends to the figures. If it is advisory to reduce the concentration of these agents which may cause side effects in the patient, after treating the cells "ex vivo", non-bound agents may be washed out from the cells by conventional techniques. This procedure widely reduces unwanted side effects caused by unbound agents within the body of the recipient.

An "effective dose" useful for treating and/or preventing these diseases or disorders may be determined using methods known to one skilled in the art (see for example, Fingl et al., The Pharmocological Basis of Therapeutics, Goodman and Gilman, eds. Macmillan Publishing Co., New York, pp. 1-46 ((1975)). This dose is preferably administered to the patient by intravenous (i.v.) or intradermal (i.d.) administration. In a preferred embodiment, ligand (b) is administered in combination with IFN-γ, e.g., in the range of 0,01 - 3 x 10⁶ IU/vaccination.

The present invention also relates to the use of a ligand to TLR2 for the preparation of a pharmaceutical composition for preventing, inhibiting or treating an infection or a proliferative disease. TLR2 and TLR1 cotranslationally form heterodimeric complexes on the cell surface and in the cytosol. Thus, a preferred ligand is a ligand that also binds to TLR1. TLR2 and TLR6 also form heterodimeric complexes. Thus, a further preferred ligand is a ligand that also binds to TLR6.

Patients having an infectious or a proliferative disorder can be treated by sequential application of the ligands, e.g., by the i.v. or i.d. route. Thus, the present invention relates to the use of (a) a ligand to TLR2 and (b) a ligand to TLR4 for the preparation of a pharmaceutical composition for the treatment of a proliferative disease requiring the sequential administration of ligands (a) and (b). Sequential administration means that ligand (a) is administered prior to ligand (b). The time span between administration of ligand (a) and (b) is preferably 1 - 48 h, more preferably 6 - 18 h.

Alternatively, patients having an infectious or a proliferative disorder can be treated by application of DC, preferably DC derived from monocytes (MoDC), that had been treated with the above ligands such that Th1 polarisation occurs. Such DC can be obtained by culturing a (highly purified) preparation of iDC in an appropriate medium, where ligand (a) and, subsequently, ligand (b) is added. Preferred concentrations of LP (OspA-LP, FSL-1LP, FSL-2LP) are 0,001 nM to 1000 nM, for LPS R60 0,001 - 1000 ng/ml. The time span between administration of ligand (a) and (b) is preferably 1 - 48 h, more preferably 6 - 18 h. Thus, the present invention also relates to a method for the in vitro-generation of Th1-polarising MoDC comprising the sequential treatment of iMoDC with (a) a ligand to TLR2 and (b) a ligand to TLR4 in a serum free medium or a medium containing human serum. Preferably, in addition to ligand (b) IFN-γ is added to the medium in a preferred concentration in the range of 100 to 2500 U/ml with a concentration of about 500 U/ml being particularly preferred.

The present invention also provides a pharmaceutical composition containing Th1-polarising MoDC that are obtainable by the method described above.

The present invention also relates to the use of Th1-polarising DC that are obtainable by the above method for the preparation of a pharmaceutical composition for preventing, inhibiting or treating infections or proliferative diseases.

Ligands to TLR2 are are lipopeptides such as the *Neisseria* outer membrane protein, PorB, a lipopeptide from *Mycoplasma salivarium,* FSL-1, a 43-kDa surface lipoprotein of *Mycoplasma fermentans,* M161Ag, an artificial modification of FSL-1, FSL-2, OspA-Lp etc. Lipopeptides are triacyl-lipopeptides or a diacyl-lipopeptides, e.g., FSL-1, FSL-2, or OspA-Lp.

Ligands to TLR4 are lipopolysaccharides (LPS). Examples of LPS that are suitable for the purposes of the present invention are LPS from *Salmonella,* with LPS R60 from an R-strain of *Salmonella minnesota* being particularly preferred. Also preferred are LPS that are particularly effective in the presence of IFN-γ.

A preferred proliferative disease that can be treated according to the present invention is cancer, preferably carcinoma, sarcoma and lymphoma.

Preferred infections are those against which no protection by a prophylactic active vaccination can be provided, such as tropical diseases. Further preferred infections that can be treated are bacterial infections, in particular those which have been shown to be refractory against antibiotic therapy such as osteomyelitis, inflammatory mammary carcinoma, abscess and others, or viral infections, in particular those which are refractory against antiviral drugs, such as hepatitis-C, virally induced meningitis, and others.

The invention is illustrated by the following examples.

### Example 1

### Materials

### (A) Lipopeptides

**FSL-1,** a lipopeptide deriving from *Mycoplasma salivarium* is available as a synthetic compound. FSL-1 is a ligand of TLR2 and TLR6 (Okusawa et al. (2004), Infect Immun 72 (3): 1657-65). It induces a down-regulation of IL-10-secretion of immature DC as measured in the ELISPOT. No effect is seen on the production of IL-12. Maturation of DC will be induced incompletely (Nishiguchi et al. (2001), J Immunol 166 (4): 2610-6).

**M161Ag** is a 43-kDa surface lipoprotein of *Mycoplasma fermentans,* serving as a potent cytokine inducer for monocytes/macrophages, maturing dendritic cells (DC), and activating host complement on affected cells. It possesses a unique N-terminal lipo-amino acid, S:-diacylglyceryl cysteine.

The 2-kDa macrophage-activating lipopeptide-2 **(MALP-2)**, recently identified as a ligand for Toll-like receptor 2 (TLR2), is derived from M161Ag. In the study resulting in the present invention, structural motifs sustaining the functions of M161Ag were identified using wild-type and unlipidated rM161Ag with (SP(+)) or without signal peptides (SP(-)). Because the SP(+) rM161Ag formed dimers via 25Cys, a monomeric form was obtained by mutagenesis (SP(+)C25S). Only wild type accelerated maturation of human DC as determined by the CD83/86 criteria, suggesting the importance of the N-terminal fatty acids for this function (Kiura et al. (2006), FEMS Immunol Med Microbiol 46 (1): 78-84).

**FSL-2** is an artificial modification of the natural FSL-1. The effects are comparable.

**OspA-Lipopeptid (OspA-Lp),** lipopeptide deriving from the outer surface protein **A** of *Borrelia burgdorferi* binds to TLR1/2 exerting similar effects as FSL-1 (Morr et al. (2002), Eur J Immunol 32(12): 3337-47).

### (B) Lipopolysaccharides (LPS)

**LPS** from an R-stem of *Salmonella minnesota* (R60) has been known to be particularly effective when acting in the presence of IFN-γ. Its effect focuses on an increase of IL-12 without affecting the synthesis of IL-10.

### Example 2

### (A) Experimental procedure

Human peripheral blood monocytes were prepared by various well-established techniques.
(1) Blood drawing was carried out in the presence of the anticoagulant EDTA, separation of the nucleated cells by combined density/velocity gradient centrifugation, followed by selective adherence in the culture for one hour. The non-attached cells were washed off and the remaining population of highly enriched monocytes was further cultured.
(2) Alternatively, blood cells were recovered by leukapheresis, followed by counterflow elutriation. The highly enriched monocyte fraction was either freeze protected or taken into culture. Cells were cultured in a humidified incubator (37°C, 5% CO₂). Medium: RPMI 1640 plus serum. CellGro DC (CellGenix) were used for serum-free culture.

### (B) iDC generation

Cytokines GM-CSF (800 IU/ml; Bayer Healthcare Pharmaceutical) plus GMP-certified IL-4 (500 IU/ml; CellGenix, Freiburg, Germany) were added to induce transdifferentiation from monocytes to immature monocyte-derived dendritic cells (iMoDC). After 5-7 days of culture iMoDC were analysed for the surface markers CD1a, CD209 und CD14 using the antibodies listed in the following table.

**Table**

| **CD-Cluster** | **fluorochrome** | **clone** | **Source** | **Ig-type** |
|---|---|---|---|---|
| CD1a | FITC | HI149 | BD-Pharmingen | IgG1 |
| CD1a | Pc5 | BL6 | BeckmanCoulter | IgG1 |
| CD14 | FITC | RMO52 | BeckmanCoulter | IgG2a |
| CD14 | Pc5 | RMO52 | BeckmanCoulter | IgG2a |
| CD209 | PE | AZND1 | BeckmanCoulter | IgG1 |
| CD86 | FITC | 2331(FUN-1) | BD-Pharmingen | IgG1 |
| CD86 | PE | HA5.2B7 | BeckmanCoulter | IgG2b |
| CD80 | PE | L307.4 | BD-Pharmingen | IgG1 |
| CD83 | PE | HB15A | BeckmanCoulter | IgG2b |
| CD83 | Pc5 | HB15A | BeckmanCoulter | IgG2b |

Suspended DC were removed from the attached fraction of macrophage-like variants. The suspended cells were either primed with an antigen and/or further treated in the described manner (see below).

### (C) Maturation culture

Maturation signals were added, including signals to trigger polarisation. The cells were further cultured for another 2 days. Thereafter the cells were analysed for the surface markers CD83, CD80, CD86, CD1a, CD209 using antibodies listed in the table, above.

### (D) Maturation culture in two steps

Alternatively, the maturation phase was split into individual steps:
Step 1: TLR agonists lipopeptides
Step 2: presence of the previous inducers, TLR agonists lipopolysaccharide added in the presence of IFN-γ.

### (E) Indicator culture

This culture imitates the fate of DC after injecting them into an organism and is designed to test the actual production and release of IL-12 and IL-10 during this phase. This is tested by the well-known ELISPOT-assay. For this phase the previous inducers were either sustained or washed out. Also, further inducers were added. Number of spots as well as the intensity of spots could be read out. Therefore, this setup does more realistically reflect the actual functional state of DC at the time of injection and thereafter. This contrasts to measuring the released cytokines in prior phases of culture, a procedure that does not tell, whether the production of cytokines has been exhausted or sustained.

### Example 3

### Stimulation of iMoDC by Lipopeptides

Treatment of iMoDC with non-toxic concentrations of lipopeptides (OspA-Lp, FSL-1-LP, FSL-2-LP available from EMC microcollection GmbH, Tübingen, Germany; see Figure 1) during 20-48 hours led to modification of cell phenotype (Figure 2) and decrease of IL-10 secreting cells in a dose-dependent (Figure 4), and donor-independent fashion (Figure 5). Costimulatory signals (CD80, CD86) were upregulated. No complete maturation has been induced, as measured by expression of CD83 (Figure 3). Although IL-10 production of MoDC is downregulated by LP-treatment, IL-12p70 production is only poorly stimulated (Figure 6). For further details, see also legends to Figures 1 to 6.

### Example 4

### Stimulation of iMoDC by LPS R60 + IFN-γ

Stimulation of iMoDC by LPS R60 +IFN-γ induces maturation with upregulation of CD83 (see also Figure 10) and induction of IL-12p70 production but not necessarily downregulation of IL-10 production (Figure 7). For further details, see also legends to Figures 7 and 10.

### Example 5

### Combination of the treatments of Examples 3 and 4

If treatment of Example 3 is combined with treatment of Example 4 in a sequential manner and in this sequence, the IL-12/IL-10 ratio increases (Figures 8 and 9). At the same time complete maturation of DC is induced as determined by upregulation of costimulatory molecules CD80 and CD86 as well as the maturation marker CD83 (Figure 10).

If the components of Examples 3 and 4 are added simultaneously or in an inverted sequence or with a reduced time interval between the two treatments, the IL-12/IL-10 ratio is reduced (Figure 9)

## Claims

1. Pharmaceutical composition for the sequential administration of (a) a ligand to TLR2 prior to (b) a ligand to TLR4 containing (a) a triacyl-lipopeptide or a diacyl-lipopeptide as a ligand to TLR2 and (b) lipopolysaccharide (LPS) as a ligand to TLR4 as separate entities.

2. The pharmaceutical composition of claim 1, wherein said pharmaceutical composition further contains IFN-γ (for the administration together with the lipopolysaccharide in step (b)).

3. A ligand to TLR2 and a ligand to TLR4 for the treatment of an infection or a proliferative disease, wherein (a) the ligand to TLR2 is a triacyl-lipopeptide or a diacyl-lipopeptide and (b) the ligand to TLR4 is a lipopolysaccharide and (a) the triacyl-lipopeptide or the diacyl-lipopeptide is sequentially administered prior to (b) the lipopolysaccharide.

4. Method for the ex-vivo generation of Th1-polarising MoDC comprising the sequential treatment of iMoDC with (a) a triacyl-lipopeptide or a diacyl-lipopeptide as a ligand to TLR2 prior to (b) a lipopolysaccharide (LPS) as a ligand to TLR4 in a serum free medium or a medium containing human serum.

5. Pharmaceutical composition containing Th1-polarising MoDC that are obtained by the method of claim 4.

6. Th1-polarising MoDC that are obtained by the method of claim 4 for use in preventing, inhibiting or treating an infection or a proliferative disease.

7. The method of claim 4, wherein in addition to the lipopolysaccharide IFN-γ is added to the medium.

8. The pharmaceutical composition of claim 1, 2 or 5, wherein said lipopeptide is FSL-1, FSL-2, or OspA-Lp.

9. The pharmaceutical composition of claim 1, 2 or 5, wherein said lipopolysaccharide (LPS) is from Salmonella.

10. The pharmaceutical composition according to claim 9, wherein said LPS is LPS R60.

11. The method according to claim 4 or 7, wherein said lipopeptide is FSL-1, FSL-2, or OspA-Lp.

12. The method according to claim 4 or 7, wherein said LPS is LPS R60.

13. The Th1-polarising MoDC according to claim 6 for use in preventing, inhibiting or treating of cancer.

14. The Th1-polarising MoDC according to claim 13, wherein said cancer is a carcinoma, sarcoma and lymphoma.

15. The Th1-polarising MoDC according to claim 6 for use in preventing, inhibiting or treating an infection, wherein said infection is a tropical disease, a bacterial or viral infection caused by infectious agents against which no prophylactic vaccination is available or which are refractory against drug treatment.

## Patentansprüche

1. Pharmazeutische Zusammensetzung zur sequentiellen Verabreichung von (a) einem Bindungsprotein für TLR2 vor (b) einem Bindungsprotein für TLR4, enthaltend (a) ein Triacyl-Lipopeptid oder Diacyl-Lipopeptid als Bindungsprotein für TLR2 und (b) ein Lipopolysaccharid (LPS) als Bindungsprotein für TLR4 als separate Einheiten.

2. Pharmazeutische Zusammensetzung nach Anspruch 1, wobei die pharmazeutische Zusammensetzung darüber hinaus IFN-γ (zur Verabreichung zusammen mit dem Lipopolysaccharid in Schritt (b)) enthält.

3. Bindungsprotein für TLR2 und Bindungsprotein für TLR4 zur Behandlung einer Infektion oder einer proliferativen Krankheit, wobei (a) das Bindungsprotein für TLR2 ein Triacyl-Lipopeptid oder Diacyl-Lipopeptid ist und (b) das Bindungsprotein für TLR4 ein Lipopolysaccharid ist und (a) das Triacyl-Lipopeptid oder das Diacyl-Lipopeptid vor (b) dem Lipopolysaccharid sequentiell verabreicht wird.

4. Verfahren zur Ex-vivo-Erzeugung von Th1-polarisierenden MoDC, umfassend die sequentielle Behandlung von iMoDC mit (a) einem Triacyl-Lipopeptid oder Diacyl-Lipopeptid als Bindungsprotein für TLR2 vor (b) einem Lipopolysaccharid (LPS) als Bindungsprotein für TLR4 in einem serumfreien Medium oder einem Medium, das Humanserum enthält.

5. Pharmazeutische Zusammensetzung, die Th1-polarisierende MoDC enthält, die durch das Verfahren nach Anspruch 4 gewonnen werden.

6. Th1-polarisierende MoDC, die durch das Verfahren nach Anspruch 4 gewonnen werden, zur Verwendung bei der Verhinderung, Hemmung oder Behandlung einer Infektion oder proliferativen Krankheit.

7. Verfahren nach Anspruch 4, wobei dem Medium zusätzlich zum Lipopolysaccharid noch IFN-γ zugesetzt wird.

8. Pharmazeutische Zusammensetzung nach Anspruch 1, 2 oder 5, wobei es sich bei dem Lipopeptid um FSL-1, FSL-2 oder OspA-Lp handelt.

9. Pharmazeutische Zusammensetzung nach Anspruch 1, 2 oder 5, wobei das Lipopolysaccharid (LPS) Salmonellen entstammt.

10. Pharmazeutische Zusammensetzung nach Anspruch 9, wobei es sich bei dem LPS um LPS R60 handelt.

11. Verfahren nach Anspruch 4 oder 7, wobei es sich bei dem Lipopeptid um FSL-1, FSL-2 oder OspA-Lp handelt.

12. Verfahren nach Anspruch 4 oder 7, wobei es sich bei dem LPS um LPS R60 handelt.

13. Th1-polarisierende MoDC nach Anspruch 6, zur Verwendung bei der Verhinderung, Hemmung oder Behandlung einer Krebserkrankung.

14. Th1-polarisierende MoDC nach Anspruch 13, wobei es sich bei der Krebserkrankung um ein Karzinom, Sarkom oder Lymphom handelt.

15. Th1-polarisierende MoDC nach Anspruch 6, zur Verwendung bei der Verhinderung, Hemmung oder Behandlung einer Infektion, wobei die Infektion eine Tropenkrankheit, eine bakterielle oder virusbedingte Infektion ist, die durch Infektionserreger verursacht wird, für die keine prophylaktische Impfung verfügbar ist oder bei der eine medikamentöse Behandlung nicht anschlägt.

## Revendications

1. Composition pharmaceutique pour l'administration séquentielle de : a) un ligand au TLR2 avant ; b) un ligand au TLR4 contenant : a) un triacyl-lipopeptide ou un diacyl-lipopeptide comme ligand au TLR2 ; et b) un lipopolysaccharide (LPS) comme ligand au TLR4 sous forme d'entités séparées.

2. Composition pharmaceutique selon la revendication 1, dans laquelle ladite composition pharmaceutique contient en outre de l'IFN-γ (pour l'administration avec le lipopolysaccharide à l'étape b)).

3. Ligand au TLR2 et ligand au TLR4 pour le traitement d'une infection ou d'une maladie proliférative, dans lesquels : a) le ligand au TLR2 est un triacyl-lipopeptide ou un diacyl-lipopeptide ; et b) le ligand au TLR4 est un lipopolysaccharide ; et a) le triacyl-lipopeptide ou le diacyl-lipopeptide est administré séquentiellement avant b) le lipopolysaccharide.

4. Procédé pour la génération ex-vivo de MoDC polarisant le Th1, comprenant le traitement séquentiel de iMoDC avec : a) un triacyl-lipopeptide ou un diacyl-lipopeptide comme ligand au TLR2 avant ; b) un lipopolysaccharide (LPS) comme ligand au TLR4 dans un milieu sans sérum ou un milieu contenant du sérum humain.

5. Composition pharmaceutique contenant des MoDC polarisant le Th1, obtenus par le procédé selon la revendication 4.

6. MoDC polarisant le Th1, obtenus par le procédé selon la revendication 4, destinés à être utilisés pour la prévention, l'inhibition ou le traitement d'une infection ou d'une maladie proliférative.

7. Procédé selon la revendication 4, pour lequel, en plus du lipopolysaccharide, de l'IFN-γ est ajouté au milieu.

8. Composition pharmaceutique selon la revendication 1, 2 ou 5, dans laquelle ledit lipopeptide est un FSL-1, un FSL-2 ou un OspA-Lp.

9. Composition pharmaceutique selon la revendication 1, 2 ou 5, dans laquelle ledit lipopolysaccharide (LPS) provient d'une salmonella.

10. Composition pharmaceutique selon la revendication 9, dans laquelle ledit LPS est un LPS R60.

11. Procédé selon la revendication 4 ou 7, pour lequel ledit lipopeptide est un FSL-1, un FSL-2 ou un OspA-Lp.

12. Procédé selon la revendication 4 ou 7, pour lequel ledit LPS est un LPS R60.

13. MoDC polarisant le Th1 selon la revendication 6, destinés à être utilisés pour la prévention, l'inhibition ou le traitement d'un cancer.

14. MoDC polarisant le Th1 selon la revendication 13, dans lesquels ledit cancer est un carcinome, un sarcome ou un lymphome.

15. MoDC polarisant le Th1 selon la revendication 6, destinés à être utilisés pour la prévention, l'inhibition ou le traitement d'une infection, dans lesquels ladite infection est une maladie tropicale, une infection bactérienne ou virale provoquée par des agents infectieux, contre lesquels aucune vaccination prophylactique n'est disponible ou qui sont réfractaires à un traitement médicamenteux.
